Europäisches Patentamt

European Patent Office

Office européen des brevets

(19)

(11) Numéro de publication: **0 069 031**
**A1**

# (12) DEMANDE DE BREVET EUROPEEN

(21) Numéro de dépôt: **82420078.6**

(22) Date de dépôt: **17.06.82**

(51) Int. Cl.³: **A 61 M 5/14, A 61 B 17/12**

(30) Priorité: **18.06.81 FR 8112353**

(43) Date de publication de la demande: **05.01.83**
**Bulletin 83/1**

(84) Etats contractants désignés: **BE CH DE GB IT LI NL SE**

(71) Demandeur: **RHONE-POULENC S.A., 25, quai Paul Doumer, F-92408 Courbevoie (FR)**

(72) Inventeur: **Bouveret, Elisabeth, 21, rue Rousselet, F-75007 Paris (FR)**
Inventeur: **Farges, Jean-Paul, 17, boulevard Carnot, F-90 000 Belfort (FR)**
Inventeur: **Honiger, Jiri, 52, avenue Camelinat, F-92100 Athis-Mons (FR)**
Inventeur: **Malbrancq, Jean-Michel, 7, rue Jeanne d'Arc, F-94320 Thiais (FR)**

(74) Mandataire: **Vogt, Bernard et al, RHONE-POULENC RECHERCHES Centre de Recherches de Saint-Fons Service Brevets B.P. 62, F-69190 Saint-Fons (FR)**

(54) **Dispositif et procédé pour faciliter la circulation sanguine extracorporelle d'un être vivant.**

(57) Dispositif pour faciliter la circulation extracorporelle d'une partie du sang d'un être vivant, caractérisé en ce qu'il comprend des moyens permettant de comprimer une partie de la circonférence d'un membre dudit être vivant, sur une certaine longueur dudit membre, de manière à concentrer le courant sanguin veineux périphérique du membre dans au moins une veine (12) située dans la partie de la circonférence du membre non comprimée par le dispositif, au moins le prélèvement du sang se faisant sur la veine non comprimée.

Procédé dans lequel on comprime une partie de la circonférence du bras d'un patient de manière à concentrer le courant sanguin veineux périphérique dans une veine non comprimée à partir de laquelle on fait le prélèvement de sang et de façon avantageuse dans laquelle on réinjecte le sang après qu'il ait circulé à l'extérieur du patient.

Application du dispositif et du procédé notamment pour la plasmaphérèse.

1

# DISPOSITIF ET PROCEDE POUR FACILITER LA CIRCULATION SANGUINE EXTRACORPORELLE D'UN ETRE VIVANT

La présente invention concerne un dispositif pour faciliter la circulation extracorporelle d'une partie du sang d'un être vivant. Plus précisément le dispositif selon la présente invention est spécialement adopté pour toutes circulations extracorporelles veino-veineuses.

Lorsqu'il est question de circulation extracorporelle veino-veineuse, on opère la plupart du temps en utilisant un garrot complet classique, constitué par une bande de caoutchouc, que l'on serre autour d'un membre, généralement sur le bras de l'être vivant (du patient) et on prélève le sang dans une veine, gonflée, à un endroit situé entre le garrot et l'extrémité du membre. Le plus généralement la ponction se fait dans la pliure du coude. La réinjection quant à elle se fait soit sur l'autre bras non garroté, soit en amont du garrot, c'est-à-dire dans la partie non située entre le garrot et l'extrémité du membre. Cette façon d'opérer, avec le garrot classique, présente deux inconvénients principaux. Le premier provient du fait qu'il n'est pas possible d'effectuer des circulations extracorporelles de longues durées avec ce garrot classique, car il y a des risques de retentions de toxines à son niveau, ce problème étant bien connu des spécialistes ; il est alors nécessaire en cours de séance de délier le garrot à intervalles pour ensuite recommencer la circulation extracorporelle. Le second inconvénient provient du fait qu'il est nécessaire avec le garrot connu classique de piquer le malade à deux endroits. Or il est connu que les patients préfèrent toujours être piqués le moins possible, d'autant plus lorsqu'ils font don d'une partie de leur sang, notamment dans le cas de plasmaphérèse par exemple.

Le but de la présente invention est donc un dispositif pour faciliter la circulation extracorporelle d'une partie du sang d'un être vivant, ledit dispositif ne présentant pas les inconvénients des dispositifs de l'art antérieur.

Il a maintenant été trouvé et c'est ce qui fait l'objet de la présente invention un dispositif caractérisé en ce qu'il comprend des moyens permettant de comprimer une partie de la circonférence d'un membre dudit être vivant, sur une certaine longueur dudit membre, de manière à concentrer le courant sanguin veineux périphérique du membre dans au moins une veine située dans la partie de la circonférence du membre non comprimée par le dispositif, au moins le prélèvement du sang se faisant sur la veine non comprimée.

La description du dispositif selon la présente invention sera mieux comprise à l'aide des figures ci-jointes qui illustrent, de façon schématique, à titre d'exemples non limitatifs, des modes de réalisation particuliers dudit dispositif.

La figure 1 représente un mode de réalisation du dispositif, sensiblement en grandeur nature (échelle 1), en coupe selon I-I de la figure 2.

La figure 2 est une vue de gauche du dispositif selon la figure 1, à échelle réduite, et simplifié.

La figure 3 représente un autre mode de réalisation du dispositif, disposé autour du bras d'un patient.

La figure 4 est une vue partielle du dispositif selon la figure 3, légèrement modifié.

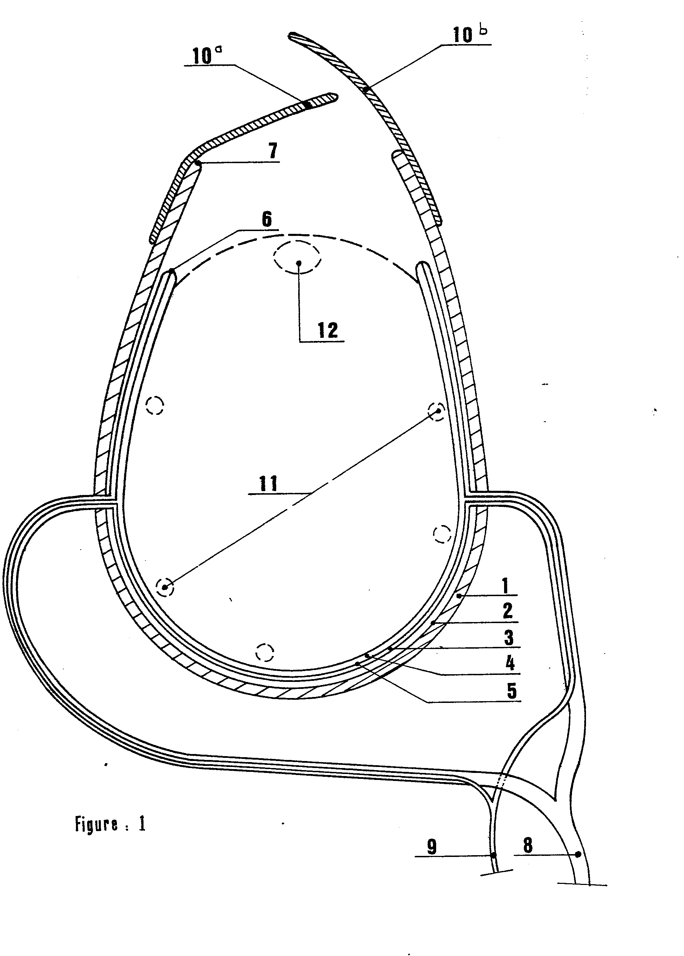

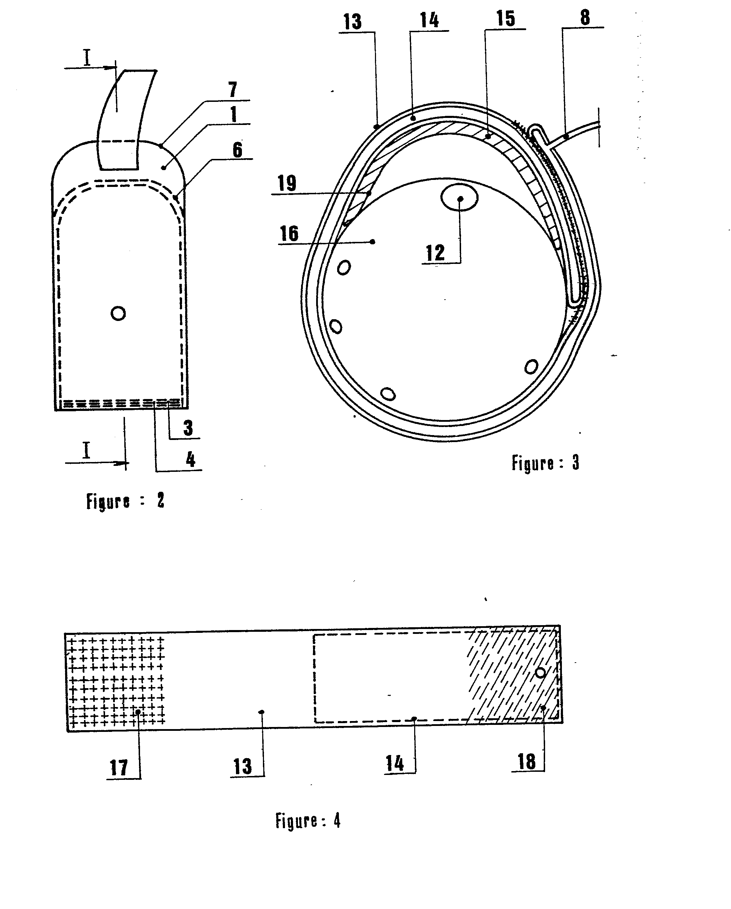

Le dispositif représenté figures 1 et 2 comprend une partie (1) de section en forme de U (appelés U parfois dans la suite de la description), dont les deux branches se rapprochent l'une de l'autre et sont avantageusement incurvées vers l'intérieur du U comme cela ressort nettement de la figure 1. A proximité de la face intérieure (2) de cette partie (1) se trouve une première poche gonflable (3), tandis qu'une seconde poche gonflable (4) se trouve contre la poche gonflable (3). Dans le cas de la figure 1 les deux poches (3 et 4) ont une paroi étanche (5) commune. Ces deux poches (3 et 4) ont sensiblement la largeur de la partie (1) comme cela est représenté plus en détail figure 2, tandis que leurs extrémités (6) n'atteignent pas les extrémités (7) des branches de la partie (1). Chaque poche gonflable est reliée à un tube souple ; la poche (3) est reliée au tube (8), la poche (4) au tube (9). Ces tubes permettent de gonfler les poches (3 et 4) grâce à des moyens connus non représentés, tels que par exemple une poire en caoutchouc, et sur ces tubes (8 et 9) sont avantageusement disposés des moyens (non représentés) pour les pincer lorsque la pression désirée est atteinte, ainsi qu'un manomètre pour mesurer cette dernière. Le dispositif selon les figures 1 et 2 comprend de plus des moyens (10a, 10b) pour maintenir les extrémités (7) des branches de la partie (1) à la distance voulue, après les avoir rapprochées lors de la mise en place du dispositif sur le patient. Ces moyens sont par exemple des bandes (10a et 10b) fixées sur chaque branche de la partie (1), ces bandes étant capables de s'agripper l'une à l'autre après avoir été mises en contact. Ce type de bandes est bien connu et est vendu sous la marque VELCRO. Chaque bande (10a et 10b) peut être fixée à la branche correspondante de la partie (1) par exemple par des rivets non représentés sur les figures 1 et 2.

Un dispositif tel que celui décrit ci-avant est de dimensions telles qu'il puisse être aisément disposé sur le bras d'un patient. Le dispositif tel que celui représenté figure 1 a été utilisé sur un patient pour une opération de plasmaphérèse, c'est-à-dire de don du plasma du sang. La partie (1) du dispositif de section en forme de U était en matière plastique, en polyméthacrylate de méthyle. Son épaisseur était de 3 mm, la distance intérieure entre les extrémités (7) des branches du U de 6 cm, la distance intérieure entre les branches du U à l'endroit où elles sont le plus éloignées de 9,5 cm, la distance extérieure entre le coude du U et les extrémités (7) de ses branches de 15 cm, la largeur de la partie (1) représentée figure 2 étant de 7 cm. Le dispositif tel que décrit est rigide, mais présente tout de même une certaine élasticité. C'est ainsi que les deux branches du U peuvent être rapprochées l'une vers l'autre jusqu'à se toucher, et elles peuvent être aisément écartées l'une de l'autre d'une distance de 10 cm environ. Les deux poches (3 et 4) gonflables étaient en caoutchouc silicone et constituées par trois bandes étanches de 0,5 mm d'épaisseur soudées l'une à l'autre sur toute leur périphérie, chaque poche étant reliée à un tube souple.

Pour la mise en oeuvre du dispositif selon la figure 1 pour une opération par exemple de plasmaphérèse, on opère de la façon suivante. On place le dispositif par exemple sur le bras d'un patient, entre son épaule et son coude, en s'arrageant pour que la partie comprise entre les deux branches du U se trouve en face ou dans l'alignement de la pliure du coude à proximité de laquelle on placera le catheter connu devant servir simultanément à la prise et à la réinjection du sang. On rapproche alors les extrémités (7) des branches du U de façon à ce que le dispositif serre un peu le bras du patient et on maintient le dispositif dans cette position en fixant les bandes (10a et 10b) l'une à l'autre. On gonfle alors la poche (3) en injectant de l'air dans le tube (8) jusqu'à ce que le patient commence à sentir une certaine pression sur son bras ; généralement on gonfle à une pression comprise entre 1 et 3 cm de mercure. On gonfle ensuite la poche (4) de la même façon en s'arrangeant pour que la pression atteinte soit inférieure à la pression artérielle diastolique, c'est-à-dire généralement inférieure à 7 ou 8 cm de mercure. Dans la figure 1, le bras du patient est représenté en section, de façon simplifiée, (la coupe des os notamment n'étant pas représentée), en trait long interrompu, tandis que ses veines sont représentées en pointillés. Il est bien entendu que lorsque le dispositif est fixé au bras, la poche gonflable (3) est en contact avec la paroi interne (2) de la partie (1) en U. Avec ce dispositif il est possible de comprimer entre les 5/10 et les 9,5/10, de préférence entre les 6/10 et les 9/10 de la circonférence du bras du patient et ainsi de concentrer le courant sanguin veineux périphérique du membre, c'est-à-dire des veines (11), dans au moins une veine (12) non comprimée. Un avantage du dispositif selon la présente invention est de pouvoir permettre la ponction et la réinjection du sang (ou d'une partie de ce dernier) dans la même veine non comprimée, généralement dans une veine superficielle du pli du coude (du patient) grâce notamment à un catheter connu de l'art antérieur qui comprend deux canaux coaxiaux, ce type de catheter étant appelé parfois catheter à double canal, ou à double lumière, ou à double flux et permettant de ne piquer qu'une seule fois le patient tout en permettant d'assurer une circulation extracorporelle. Un autre avantage du dispositif précédemment décrit résulte du fait qu'il permet d'assurer des circulations extracorporelles en continu de longue durée, par exemple de l'ordre de une heure, et avec des débits de sang relativement élevés, généralement compris entre 60 et 90 ml/mm (millilitre à la minute) selon le patient.

A titre indicatif un prélèvement fait sur un bras sans aucun garrot permet d'obtenir un débit sanguin veineux compris entre 20 et 30 ml/mm, tandis qu'un prélèvement fait avec un garrot complet classique permet d'atteindre des débits veineux de l'ordre de 60 à 100 ml/mn.

Ainsi le dispositif selon la présente invention est spécialement avantageux dans le cas de circulation extracorporelle en continu avec un catheter à double flux, car il permet de ne faire qu'une seule piqûre au patient. Mais il présente également déjà de l'intérêt par rapport au garrot complet classique, qui nécessite en cas de circulation extracorporelle de longue durée d'être défait à intervalles réguliers pour éviter le phénomène connu de rétentions de toxines.

De nombreuses variantes de réalisation du dispositif selon les figures 1 et 2 sont à la portée du technicien. Le dispositif peut par exemple n'être constitué que de la partie (1) en forme de U dans la mesure où cette dernière est par exemple en métal et présente une certaine élasticité ; la partie (1) peut ainsi être constitué par une bande d'acier mise en forme de U. A titre de variante, à l'intérieur du U peut se trouver, à la place des poches gonflables, une bande en mousse. D'autre part le dispositif selon les figures 1 et 2 peut éventuellement ne comporter qu'une poche gonflable au lieu de deux. En outre pour faciliter la mise en place du dispositif sur le bras du patient, la partie (1) peut comprendre une charnière en bas du coude du U, cette charnière permettant ainsi d'ouvrir à volonté les branches du U lors de la mise en place sur le patient. D'autre part, notamment dans ce mode de réalisation du U avec charnière, les bandes (10a et 10b) peuvent éventuellement être remplacées par un système connu d'encliquetage, un cliquet par exemple disposé sur une branche du U se positionnant sur l'autre branche du U dans des encoches spécialement prévues à cet effet et empêchant l'ouverture du U lorsque la partie (1) est placée sur le patient. Ce système d'encliquetage peut être situé à proximité des extrémités des branches du U ou vers la charnière mentionnée. Dans le cas de dispositif possédant une charnière, les deux branches du U peuvent être en matériau rigide.

Il doit être entendu d'autre part que par partie (1) en forme de U on entend également un dispositif dont la partie (1) en coupe correspond sensiblement à un anneau ouvert.

Les figures 3 et 4 représentent un autre mode de réalisation du dispositif selon la présente invention. Dans ce mode de réalisation le dispositif comprend une enveloppe (13) de forme allongée, généralement en tissu, à l'intérieur de laquelle se trouve un poche gonflable (14) également de forme allongée, reliée à un tube (8) partiellement représenté, et il comprend de plus un élément rigide (15) dont la longueur correspond à la largeur de l'enveloppe (13). La section (représentée figure 3) dudit élément (15) est telle que lorsqu'il est disposé sur le bras du patient et que l'enveloppe (13) contenant la poche gonflable (14) est enroulée autour du bras du patient, au moins une veine n'est pas comprimée par le dispositif lorsqu'on gonfle la poche (14).

Dans la figure 3 la poche gonflable (14) a sensiblement la longueur de l'enveloppe (13) mais éventuellement elle peut être de longueur inférieure comme cela est représenté figure 4. A chaque extrémité de l'enveloppe (13) se trouvent des moyens de fixation de l'enveloppe sur elle-même lorsqu'elle est disposée autour du bras (16) du patient représenté en coupe de façon simplifiée figure 3. Ainsi sur une face de l'enveloppe (13) et à une extrémité de celle-ci se trouve par exemple une première bande (17) représentée par de petites croix, tandis qu'à l'autre extrémité et sur l'autre face se trouve une seconde bande (18) représentée en hachures. Ces deux bandes (17 et 18) s'agrippent entre elles lorsqu'elles sont en contact, ce type de bandes étant commercialisées sous la marque VELCRO. On peut considérer que l'ensemble formé par la poche gonflable (14) et l'enveloppe extérieure (13) constitue une partie d'un appareil classique connu de la mesure de la tension d'un patient. L'élément (15) rigide peut être en métal ou en plastique et sa face interne (19) est avantageusement de forme concave tandis que sa face externe est convexe. Plus généralement l'élément (15) a une section telle que lorsqu'il est disposé sur le bras du patient, sa face intérieure (19), c'est-à-dire sa face regardant le bras, dans sa majeure partie ne soit pas au contact de ce dernier lorsque l'enveloppe (13) et la poche gonflable (14) sont enroulées autour du bras et dudit élément (15). Eventuellement l'élément (15) peut être fixé à l'enveloppe (13) par exemple à une de ses extrémités.

Une variante de l'appareil selon les figures 3 et 4 peut consister à remplacer l'ensemble constitué par l'enveloppe (13) et la poche gonflable (14) par une simple bande en caoutchouc, telle celle servant de façon connue à faire un garrot complet autour du bras et que l'on fixe par exemple avec une pince autour de ce dernier. Dans cette variante le dispositif selon la présente invention comprend un élément (15) et une bande de caoutchouc que l'on dispose autour du bras de la même façon que le dispositif selon la figure 3. Bien entendu dans cette variante la longueur de l'élément (15) est au moins égale à la largeur de la bande en caoutchouc.

Le dispositif selon les figures 3 et 4 présentent sensiblement les mêmes avantages que celui selon les figures 1 et 2 et peut être de même utilisé chaque fois qu'il est question de circulation extracorporelle veino-veineuse.

A titre d'exemples non limitatifs d'utilisation du dispositif selon la présente invention en circulation extracorporelle veino-veineuse, on peut citer la plasmaphérèse, c'est-à-dire le don du plasma, que ce dernier soit séparé des éléments figurés du sang (globules rouges, globules blancs, plaquettes) soit par centrifugation, soit au moyen d'appareils à membranes semi-perméables. Dans ce cas de plasmaphérèse le plasma est donné par le patient. On peut citer également la plasmaphérèse thérapeutique, c'est-à-dire l'opération par laquelle on sépare le plasma des éléments figurés du sang d'un malade et au cours de laquelle on réinjecte en continu, au même malade, en plus de ses éléments figurés, soit un autre plasma, soit son plasma après lui avoir fait subir un traitement pour en éliminer certains produits toxiques par exemple. Comme autre utilisation en circulation extracorporelle veino-veineuse du dispositif selon la présente invention, on peut également citer la leucophérèse ou cytophérèse, au cours de laquelle on isole les globules blancs du sang d'un patient. Le dispositif selon la présente invention peut être également utilisé en circulation extracorporelle lors de séances d'hémodialyse à relativement faibles débits (inférieurs à 100 ml/mn) et de façon plus générale chaque fois qu'il est question d'épuration de sang.

Il doit être signalé que lors de la mise en place du dispositif selon la présente invention, la veine dans laquelle on introduit le cathéter n'est pas gonflée comme c'est le cas lorsqu'on utilise un garrot complet classique. De ce fait, dans certains cas, il peut être difficile

8

d'introduire le cathéter et c'est pour celà qu'il peut être avantageux, après avoir mis le dispositif selon la présente invention en place sur le patient, de disposer ensuite un garrot classique complet, juste pour faciliter l'introduction du cathéter dans la veine. Le garrot complet est alors desserré et seul reste en fonctionnement le dispositif selon la présente invention au cours de la circulation extracorporelle veino-veineuse. Ainsi le dispositif selon la présente invention peut éventuellement comprendre, en plus, des moyens permettant de comprimer la totalité de la circonférence du membre du patient pour faciliter l'introduction dans la veine du cathéter devant servir à la circulation extracorporelle. A titres d'exemples ces moyens peuvent être tout simplement une bande en caoutchouc, telle celle utilisée pour la réalisation de garrot complet classique, ladite bande étant solidaire du dispositif selon les figures 1 et 2, à l'extérieur de la partie (1) en U, par exemple vers le coude du U, ou solidaire du dispositif selon les figures 3 et 4, vers l'extrémité de l'enveloppe allongée (13) comportant les moyens de fixation (17).

9

REVENDICATIONS

1. - Dispositif pour faciliter la circulation extracorporelle d'une partie du sang d'un être vivant, caractérisé en ce qu'il comprend des moyens permettant de comprimer une partie de la circonférence d'un membre dudit être vivant, sur une certaine longueur dudit membre, de manière à concentrer le courant sanguin veineux périphérique du membre dans au moins une veine située dans la partie de la circonférence du membre non comprimée par le dispositif, au moins le prélèvement du sang se faisant sur la veine non comprimée.

2. - Dispositif selon la revendication 1, caractérisé en ce qu'il comprend une enveloppe (13) de forme allongée, une poche gonflable (14) de forme allongée et un élément rigide (15) dont la longueur correspond à la largeur de la poche gonflable (14), ledit élément (15) permettant de ne pas comprimer, sous sa face intérieure (19) le membre autour duquel sont disposées l'enveloppe (13) allongée et la poche gonflable (14) qui compriment le membre.

3. - Dispositif selon la revendication 2, caractérisé en ce que l'enveloppe (13) allongée et la poche gonflable (14) constituent une partie de l'appareil classique de mesure de la tension d'un patient et recouvrent totalement l'élément rigide (15).

4. - Dispositif selon la revendication 1, caractérisé en ce qu'il comprend un garrot classique, constitué par une bande de caoutchouc, disposé fixement autour du membre et un élément (15) rigide dont la majeure partie de la face (19) intérieure n'est pas au contact dudit membre et dont la longueur correspond au moins à la largeur de la bande de caoutchouc.

5. - Dispositif selon l'une quelconque des revendications 2, 3 ou 4, caractérisé en ce que l'élément (15) rigide à sa face intérieure (19) concave.

6. - Dispositif selon la revendication 1, caractérisé en ce qu'il comprend au moins une partie (1) dont la section a la forme d'un U dont les branches sont avantageusement rapprochées l'une de l'autre vers leurs extrémités.

7. - Dispositif selon la revendication 6, caractérisé en ce qu'il comprend des moyens (10a, 10b) pour régler le rapprochement des

extrémités des branches du U lors de sa mise en place et pour maintenir constante la distance entre les deux branches du U, après que le dispositif ait été placé sur le membre de l'être vivant.

8. - Dispositif selon l'une quelconque des revendications 6 ou 7, caractérisé en ce qu'à l'intérieur de la partie (1) en forme de U se trouve une bande en matériau souple.

9. - Dispositif selon l'une quelconque des revendications 6 ou 7, caractérisé en ce qu'à l'intérieur de la partie (1) en forme de U se trouve au moins une poche (3) gonflable.

10. - Dispositif selon la revendication 9, caractérisé en ce qu'à l'intérieur de la partie (1) en forme de U se trouvent deux poches gonflables superposées (3 et 4).

11. - Dispositif selon l'une quelconque des revendications précédentes, caractérisé en ce qu'il permet de comprimer entre les 5/10 et les 9,5/10 de la circonférence du membre.

12. - Dispositif selon l'une quelconque des revendications précédentes, caractérisé en ce qu'il comprend de plus des moyens permettant de comprimer la totalité de la circonférence du membre pour faciliter l'introduction du cathéter devant servir au prélèvement.

13. - Procédé pour faciliter la circulation extracorporelle d'une partie du sang d'un être vivant, caractérisé en ce qu'on comprime une partie de la circonférence d'un membre d'un être vivant, sur une certaine longueur, de manière à concentrer le courant sanguin veineux périphérique dans au moins une veine périphérique dudit membre située dans la partie non comprimée du membre, au moins le prélèvement du sang se faisant sur ladite veine non comprimée.

14. - Procédé selon la revendication 12, caractérisé en ce que le prélèvement du sang et le retour d'au moins une partie du sang se font dans le même veine et sensiblement au même endroit grâce à un cathéter de type connu permettant de ne piquer le patient qu'une seule fois.

15. - Procédé selon l'une quelconque des revendications 13 et 14, caractérisé en ce qu'on comprime entre 0,5 et 0,95 de la circonférence du membre.

16. - Procédé selon l'une quelconque des revendications 13 à 15, caractérisé en ce qu'on comprime le membre à une pression inférieure à la pression diastolique artérielle.

17. - Procédé selon l'une des revendications 13 à 16, caractérisé en ce qu'on comprime le membre à une pression comprise entre 3 et 8 cm de mercure.

18. - Utilisation du dispositif selon l'une quelconque des revendications 1 à 12 pour une opération de plasmaphérèse.

0069031

PL. I-2

Figure : 1

PL. II-2

Figure : 2

Figure : 3

Figure : 4

0069031

Office européen
des brevets

**RAPPORT DE RECHERCHE EUROPEENNE**

Numéro de la demande

EP  82 42 0078

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| X | FR-A-1 003 251  (J. ROOS)<br><br>* figure 1; colonne 2, page 1, lignes 4,5; colonne 1, page 1, lignes 40,41; colonne 2, page 1, lignes 1,2,3 *<br><br>--- | 1,11, 13,14, 16 | A 61 M   5/14<br>A 61 B  17/12 |
| X,P | US-A-4 314 568  (J.A. LOVING)<br><br>* figures 1,2; colonne 1, lignes 13,14; colonne 1, lignes 19,20; colonne 2, lignes 64-69; colonne 3, ligne 1; colonne 3, lignes 43-45 *<br><br>--- | 1,11, 13,14, 16 | |
| A | GB-A-1 400 957  (R. ARONSON)<br><br>* figures 1,3; page 1, lignes 13-19; page 4, lignes 56-64 *<br><br>----- | 2,3,9, 10 | **DOMAINES TECHNIQUES RECHERCHES (Int. Cl. ³)**<br><br>A 61 M<br>A 61 B |

Le présent rapport de recherche a été établi pour toutes les revendications

| Lieu de la recherche | Date d'achèvement de la recherche | Examinateur |
|---|---|---|
| LA HAYE | 16-09-1982 | HARRISON M.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82